# EUROPEAN PATENT SPECIFICATION

(11) **EP 4 057 288 B1**
(45) Date of publication and mention of the grant of the patent: **06.09.2023**
(21) Application number: 22161733.5
(22) Date of filing: 11.03.2022
(51) Int. Cl.: G16H 10/60, G16H 40/63

(54) **ENHANCED REPORTING AND CHARTING OF VITAL SIGNS AND OTHER PATIENT PARAMETERS**
VERBESSERTES BERICHTEN UND AUFZEIGEN VON VITALZEICHEN UND ANDEREN PATIENTENPARAMETERN
ÉTABLISSEMENT AMÉLIORÉ DE RAPPORTS ET DE GRAPHIQUES DE SIGNES VITAUX ET D'AUTRES PARAMÈTRES DE PATIENT

(30) Priority: 12.03.2021 US 202163160632 P
(43) Date of publication of application: 14.09.2022
(73) Proprietor: Welch Allyn, INC., Skaneateles Falls, NY 13153-0220 (US)
(72) Inventor: BERGSTROM, Jennifer, Skaneateles Falls, 13153-0220 (US); BROUGH, Stacie L, Skaneateles Falls, 13153-0220 (US); MOON, Tiffany L, Skaneateles Falls, 13153-0220 (US); WILLIAMSON, Rachel L, Skaneateles Falls, 13153-0220 (US); YEUNG, Ching Yue, Skaneateles Falls, 13153-0220 (US); ROBERTS, Chris R, Skaneateles Falls, 13153-0220 (US); McSWEENEY, WonKyung, Skaneateles Falls, 13153-0220 (US); LONG, Christopher, Skaneateles Falls, 13153-0220 (US); DEBRAH, Dan, Skaneateles Falls, 13153-0220 (US)
(74) Representative: Findlay, Alice Rosemary

(56) References cited:
- US-A1- 2014 188 516
- US-A1- 2015 363 563
- US-A1- 2018 214 091

## Description

This application relates generally to aggregating patient parameters detected by multiple sensors, as well as techniques for updating electronic medical records (EMRs). Additionally, this application relates generally to techniques for generating customized control charts.

In a clinical ward environment, a patient may be monitored using multiple different sensors. For example, the oxygenation level of the patient's blood may be monitored by a finger-clip pulse oximetry sensor, the blood pressure of the patient may be monitored by a blood pressure cuff, and so on. In general, these sensors do not communicate. Thus, a care provider may review the condition of the patient by utilizing each one of the different sensors, individually.

In addition, a care provider may update the EMR of the patient based on the parameters output by the different sensors. In general, a care provider may access a web portal associated with the EMR of the patient and may manually enter the parameters detected by the individual sensors. For instance, a care provider may write down the parameters output by the individual sensors, and then enter the parameters into a computing device that interfaces with one or more servers storing the EMR of the patient.

Moreover, vital signs of the patient can vary based on many factors including gender, age, etc. Accordingly, alarms associated with different sensors monitoring the patient may be triggered due to standardized alarm settings, resulting in alarm fatigue.

US2018/214091A1 discloses a patient monitoring system including sensors, a patient support assembly, and a gateway device and/or control module. Example sensors include physiological sensors and patient status devices. The sensors acquire patient-related data. Patient related action is determined based on the patient-related data.

US2015/363563A1 discloses various exemplary methods and systems for automated deployment of remote measurement, patient monitoring, and home care and multi-media collaboration services in health care and telemedicine. In general, a system can allow automated deployment and simplified installation by non-technical personnel of sensor-based remote monitoring and process automation solutions.

The present invention, according to an aspect of the present disclosure, is defined by the appended claims.

Various implementations of the present disclosure relate to presenting multiple parameters of a patient on a single monitor for review by a care provider and to providing a way for the care provider to efficiently chart the parameters of the patient in an EMR. The parameters may be detected and/or generated by multiple different sensors, including sensors integrated into a hospital bed supporting the patient. The monitor may report the parameters of the patient to the care provider simultaneously, thereby providing the care provider with valuable context into the patient's holistic condition.

Rather than automatically uploading each and every parameter as detected, the monitor may confirm the parameters before uploading them to the EMR. In various cases, the monitor may output the parameters to the care provider, who may confirm the accuracy of the parameters by providing an input signal to the monitor. By outputting multiple parameters to the care provider, simultaneously, the care provider may have a more holistic perspective of the condition of the patient and may be able to more accurately identify whether any of the parameters are erroneous. The monitor may also use other techniques to confirm the accuracy of the parameters. In some cases, the monitor may selectively upload parameters that are within physiological ranges, which may prevent the EMR of the patient from being updated based on physiologically impossible parameters. In the preferred embodiment of the invention, the monitor confirms a first parameter detected by a first sensor based on a second parameter detected by a second sensor. For example, the monitor may use a weight of the patient detected by a load cell of the hospital bed to determine that the patient is sitting upright in the hospital bed, and use that determination to conclude that the blood pressure of the patient, detected by a sensor outside of the hospital bed, is likely to be accurate. In various implementations, the monitor may discard and/or refrain from uploading inaccurate parameters to the EMR of the patient. Thus, the monitor may prevent the EMR of the patient from being updated with erroneous parameters.

In various implementations, the EMR is maintained by one or more servers that are located remotely from the clinical environment. The monitor may aggregate multiple parameters detected by multiple different sensors. Thus, the monitor may transmit the parameters to the EMR server(s) using a single transmission, rather than multiple transmissions. By aggregating the parameters into a single transmission, the monitor may reduce the burden of updating the EMR on communications resources, such as network bandwidth. Furthermore, in examples where an organization controlling the EMR may limit and/or charge for the number of transmissions to the EMR server(s), the monitor may reduce the financial burden of electronic charting.

The invention will now be further described by way of example with reference to the accompanying drawings, in which:
FIG. 1 illustrates an example environment for automatically updating an EMR with detected patient parameters.
FIG. 2 illustrates an example environment wherein parameters from multiple patients are reported to an EMR system.
FIG. 3 illustrates an example of a monitor used to aggregate, display, and report multiple parameters of a patient.
FIG. 4 illustrates an example data packet carrying EMR data.
FIG. 5 illustrates an example process for updating an EMR of an individual based on parameters of the individual.
FIG. 6 illustrates an example process for selectively reporting at least one vital sign of an individual to an EMR system.
FIG. 7 illustrates at least one example device configured to enable and/or perform the some or all of the functionality discussed herein.

Various implementations of the present disclosure will be described in detail with reference to the drawings, wherein like reference numerals present like parts and assemblies throughout the several views. Additionally, any samples set forth in this specification are not intended to be limiting and merely set forth some of the many possible implementations.

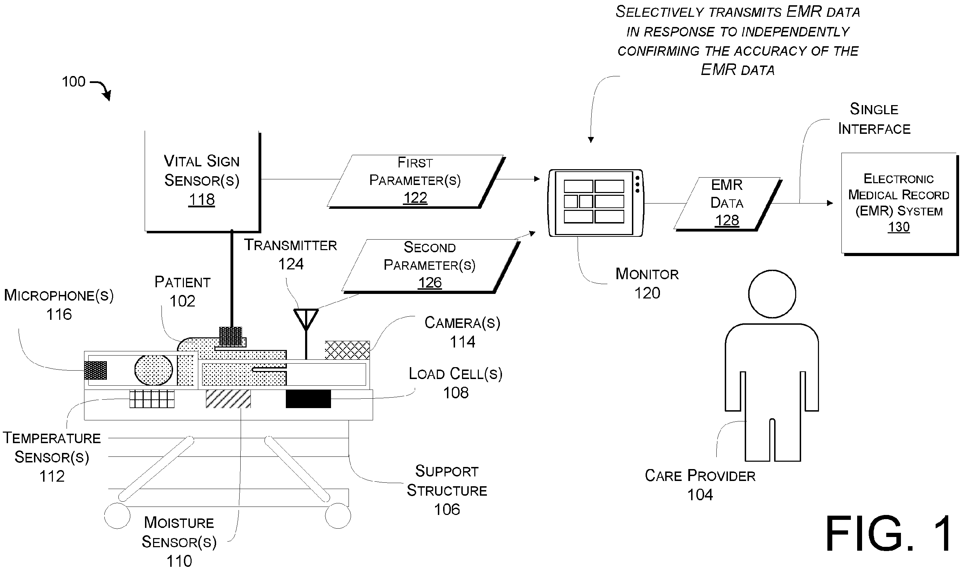

FIG. 1 illustrates an example environment 100 for automatically updating an EMR with detected patient parameters. In the environment 100, a patient 102 is monitored by a variety of sensors. The patient 102 may be any individual with a health issue that could trigger health deterioration. For example, the patient 102 may be human in an in-patient ward of a hospital.

In various examples, to avoid the potential health deterioration, or to address any deterioration quickly and efficiently, the patient 102 is monitored in a medical ward of a clinical environment. A care provider 104 may be responsible for monitoring the patient 102. The care provider 104 may be responsible for monitoring multiple patients including the patient 102, such as five to ten patients, or more, in the clinical environment. Accordingly, it may be impossible for the care provider 104 to constantly monitor the patient 102. The care provider 104, for example, may be a nurse, a physician, a physician's assistant (PA), a medical student, a nursing student, or a medical technician.

To enable the care provider 104 to monitor the patient 102 in addition to other patients within the clinical environment, a variety of sensors may be configured to detect parameters of the patient 102. As used herein, the term "parameter," and its equivalents, may refer to a metric indicative of a condition of a patient 102. Example parameters may be detected and/or measured from the patient 102. Examples of parameters include vital signs, such as respiratory rate, oxygen saturation, blood pressure, heart rate, and level of consciousness (e.g., alert, verbal, pain, unresponsive (AVPU) level). According to some examples, a parameter includes an early warning score (EWS). The term "EWS," and its equivalents, can refer to a metric based on multiple parameters. Examples of an EWS include the pediatric early warning score (PEWS), national early warning score (NEWS and NEWS2), modified early warning score (MEWS), modified early obstetric warning score (MEOWS), and so on. In some cases, parameters include movement, a position, an audio, a video, a weight, a moisture, a nutrition, an electrical signal (e.g., an electrocardiogram (ECG) or electroencephalogram (EEG)), a respiration (e.g., a capnograph or end-tidal CO₂ (EtCO₂)), a cardiac output, a blood glucose level, an albumin level, an amylase level, a calcium level, a creatinine level, an erythrocyte level, a hemoglobin level, a leukocyte level, a platelet level, a urea level, a sodium level, or a potassium level of the patient 102.

At least a portion of the sensors may be disposed on, attached to, or integrated with a support structure 106. The support structure 106 includes, for instance, a gurney, hospital bed, or some other structure configured to support the patient 102. As used herein, the terms "bed," "hospital bed," and their equivalents, can refer to a padded surface configured to support a patient for an extended period of time (e.g., hours, days, weeks, or some other time period). The patient 102 may be laying down on the support structure 106. For example, the patient 102 may be resting on the support structure 106 for at least one hour, at least one day, at least one week, or some other time period. In various examples, the patient 102 and the support structure 106 may be located in the clinical environment. In some implementations, the support structure 106 includes a mechanical component that can change the angle at which the patient 102 is disposed. In some cases, the support structure 106 includes padding to distribute the weight of the patient 102 on the support structure 106. According to various implementations, the support structure 106 itself can include vital sign monitors (e.g., the monitor 120) configured to output alarms or otherwise communicate vital signs of the patient 102 to external observers (e.g., the care provider 104, family members, and the like). The support structure 106 may include railings that prevent the patient 102 from sliding off of a resting surface of the support structure 106. The railings may be adjustable, in some cases.

In various examples, the support structure 106 includes one or more load cells 108. The load cell(s) 108 may be configured to detect a pressure on the support structure 106. In various cases, the load cell(s) 108 can include one or more strain gauges, one or more piezoelectric load cells, a capacitive load cell, an optical load cell, any device configured to output a signal indicative of an amount of pressure applied to the device, or a combination thereof. For example, the load cell(s) 108 may detect a pressure (e.g., weight) of the patient 102 on the support structure 106. In some cases, the support structure 106 includes multiple load cells 108 that respectively detect different pressures on the support structure 106 in different positions along the support structure 106. In some instances, the support structure 106 includes four load cells arranged at four corners of a resting surface of the support structure 106, which respectively measure the pressure of the patient 102 on the support structure 106 at four regions located at the four corners of the support structure 106. The resting surface, for instance, can be a surface in which the patient 102 contacts the support structure 106, such as a top surface of the support structure 106.

The support structure 106 may also include one or more moisture sensors 110. The moisture sensor(s) 110 may be configured to measure a moisture on a surface (e.g., the resting surface) of the support structure 106. For example, the moisture sensor(s) 110 can include one or more capacitance sensors, one or more resistance sensors, one or more thermal conduction sensors, or a combination thereof. In some cases, the moisture sensor(s) 110 include one or more fiber sheets configured to propagate moisture to detectors included in the moisture sensor(s) 110. In some cases, the moisture sensor(s) 110 can detect the presence or absence of moisture (e.g., sweat or other bodily fluids) disposed between the support structure 106 and the patient 102.

In various examples, the support structure 106 can include one or more temperature sensors 112. The temperature sensor(s) 112 may be configured to detect a temperature of the patient 102 and/or the support structure 106. In some cases, the temperature sensor(s) 112 includes one or more thermistors, one or more thermocouples, one or more resistance thermometers, one or more Peltier sensors, or a combination thereof.

The support structure 106 may further include one or more cameras 114. The camera(s) 114 may be configured to capture images of the patient 102, the support structure 106, an ambient environment (e.g., a room) in which the patient 102 and support structure 106 are located, or a combination thereof. In various cases, the camera(s) 114 may include radar sensors, infrared cameras, visible light cameras, depth-sensing cameras, or any combination thereof. In some examples, infrared images may indicate, for instance, a temperature profile of the patient 102 and/or the support structure 106. Thus, the camera(s) 114 may be a type of temperature sensor. In addition, the images may indicate a position of the patient 102 and/or the support structure 106, even in low-visible-light conditions. For example, the infrared images may capture a position of the patient 102 during a night environment without ambient lighting in the vicinity of the patient 102 and/or the support structure 106. In some cases, the camera(s) 114 may include one or more infrared video cameras. The camera(s) 114 may include at least one depth-sensing camera configured to generate a volumetric image of the patient 102, the support structure 106, and the ambient environment. In some examples, the camera(s) 114 capture multiple images of the patient 102, the support structure 106, and/or the ambient environment, such as a video of the patient 102, the support structure 106, and/or the ambient environment. In some cases, the camera(s) 114 are configured to capture at least one image or a video an entrance to a room containing the support structure 106, an entrance to a bathroom adjacent to the room containing the support structure 106, or a combination thereof. According to various implementations, the images and/or videos captured by the camera(s) 114 are indicative of a position and/or a movement of the patient 102 over time.

In some examples, the support structure 106 can include one or more microphones 116 configured to capture audio signals output by the patient 102, the support structure 106, and/or the ambient environment. The audio signals captured by the microphone(s) 116 may be indicative of a position and/or movement of the patient 102 over time. In some examples, the audio signals captured by the microphone(s) 116 may be indicative of an alertness, emotional state, and/or a pain level of the patient 102. For instance, if the audio signals indicate that the patient 102 is speaking clearly and laughing with family members, the audio signals may indicate that the patient 102 is relatively alert with adequate pain management. In particular cases, the microphone(s) 116 are integrated within the camera(s) 114.

In some examples, the support structure 106 also includes a head rail and a foot rail. The camera(s) 114, for instance, are mounted on the head rail, the foot rail, an extension (e.g., a metal or polymer structure) attached to the head rail or the foot rail, or any combination thereof. In various implementations, the camera(s) 114 are attached to a wall or ceiling of the room containing the support structure 106. In some examples, the camera(s) 114 are attached to a cart or other object that is located in the vicinity of the support structure 106.

The load cell(s) 108, moisture sensor(s) 110, temperature sensor(s) 112, camera(s) 114, and microphone(s) 116 are all examples of sensors configured to detect parameters of the patient 102. In addition, the patient 102 may be monitored by one or more vital sign sensors 118. The vital sign sensor(s) 118 may be integrated with the support structure 106, separate from the support structure 106, or a combination thereof. Examples of the vital sign sensor(s) 118 include a respiratory sensor (e.g., an impedance pneumography sensor, a capnography sensor, etc.), an oxygen saturation sensor (e.g., an oximeter adhered to the patient 102 or clipped on a limb of the patient 102, such as a finger, etc.), a blood pressure sensor (e.g., a blood pressure cuff, an arterial catheter, etc.), and a heart rate sensor.

According to various implementations, the sensors may detect parameters of the patient 102 and provide those parameters to a monitor 120. For instance, the vital sign sensor(s) 118 may be configured to transmit a signal indicating one or more first parameters 122 of the patient 102 to the monitor 120. Further, the support structure 106 may include a transmitter 124 configured to transmit a signal indicating one or more second parameters 126 to the monitor 120. In some cases in which the vital sign sensor(s) 118 are integrated with the support structure 106, the first parameter(s) 122 may also be transmitted to the monitor 120 by the transmitter 124. In some cases, the first parameter(s) 122 and/or the second parameter(s) 126 may be transmitted to the monitor 120 via one or more data streams. For example, the vital sign sensor(s) 118 and the transmitter 124 of the support structure 106 may transmit the first parameter(s) and the second parameter(s) 126 substantially in real-time as the first parameter(s) 122 and the second parameter(s) 126 are detected. In some cases, a latency between when a parameter is detected and when the parameter is sent to the monitor 120 is no more than 10 seconds, 5 seconds, 1 second, 100 milliseconds (ms), 10 ms, or 1 ms. For instance, the latency may be between 1 ms and 10 seconds.

The first parameter(s) 122 and the second parameter(s) 126 may be transmitted over one or more communication networks. The communication network(s) may include, for instance, at least one wired interface (e.g., an ethernet interface, an optical cable interface, etc.) and/or at least one wireless interface (e.g., a BLLTETOOTH interface, a WI-FI interface, a nearfield communication (NFC) interface, a Long Term Evolution (LTE) interface, a New Radio (NR) interface, etc.). In some cases, the first parameter(s) 122 and/or the second parameter(s) 126 are transmitted over a wide area network (WAN), such as the Internet. In some cases, the first parameter(s) 122 and/or the second parameter(s) 126 include one or more data packets (e.g., Internet Protocol (IP) data packets), datagrams, or a combination thereof.

In various implementations, the monitor 120 may include at least one computing device configured to receive the first parameter(s) 122 and the second parameter(s) 126. Examples of the monitor 120 include a personal computer, a tablet computer, a smart television (TV), a mobile device, a mobile phone, an Internet of Things (IoT) device, or another type of computing device. The monitor 120 may be configured to report the first parameter(s) 122 and/or the second parameter(s) 126 to the care provider 104. For example, the monitor 120 may include a display configured to visually output indications of the first parameter(s) 122 and/or the second parameter(s) 126. In some cases, the monitor 120 includes a speaker configured to audibly output indications of the first parameter(s) 122 and/or the second parameter(s) 126. According to some implementations, the monitor 120 includes a haptic device configured to at least partially indicate the first parameter(s) 122 and/or the second parameter(s) 126 by vibrating. According to some implementations, the monitor 120 may output multiple parameters simultaneously, which may contextualize the parameters for review by the care provider 104. For example, the monitor may output the blood pressure and heart rate of the patient 102, simultaneously, and the care provider 104 can determine whether the blood pressure reading is reasonable in view of the heart rate reading.

In some examples, the monitor 120 may include an input device (e.g., a button, a touch sensor, a keyboard, etc.) configured to receive an input signal from the care provider 104 indicative of other parameters. For example, the care provider 104 may interact with the patient 102, directly, to identify a pain level, a level of consciousness, or another type of parameter of the patient 102. The care provider 104 may enter the pain level and/or level of consciousness into the monitor 120 using the input device.

In some examples, the monitor 120 may derive a parameter based on the first parameter(s) 122 and/or the second parameter(s) 126, and output the derived parameter to the care provider 104. For example, the monitor 120 may calculate an EWS of the patient 102, a falls risk of the patient 102, a sepsis risk of the patient 102, a pressure injury risk of the patient 102, or some other derived parameter, based on the first parameter(s) 122 and/or the second parameter(s) 126. The monitor 120 may further output the derived parameter.

The term "EWS" refers to a value generated based on vital sign parameters and physical assessment metrics. The assigned values can be used to assess the patient status or perform calculations to assess the patient status. The assessment metrics in combination with one or more vital signs parameter allow the caregiver to judge whether or not a patient's condition is improving or deteriorating. For example, an EWS may drive a user action. For instance, a care provider may reference an EWS cheat sheet (e.g., a printed table) that indices recommended user actions based on the EWS of a patient. Examples of EWSs include the National Early Warning Score 2 (NEWS2) and Modified Early Warning Score (MEWS).

NEWS2, for example, can be used to estimate whether a patient requires critical care intervention. NEWS2 provides a single numerical value based on a patient's vital signs, wherein the vital signs include respiration rate, SpO₂₎ (defined according to a Scale 1 or a Scale 2, as defined according to National Health Service (NHS) guidelines), exposure to air or supplemental oxygen, systolic blood pressure, pulse, level of consciousness, and temperature. The monitor 120 can determine an additive factor for each vital sign, based on which of multiple ranges each vital sign fits into. The single numerical value can be determined by summing the additive factors together. The following Table 1 provides example additive factors corresponding to each vital sign:

**Table 1**

| **Additive Factor** | **3** | **2** | **1** | **0** | **1** | **2** | **3** |
|---|---|---|---|---|---|---|---|
| Respiration Rate (per minute) | ≤8 | | 9-11 | 12-20 | | 21-24 | ≥25 |
| SpO₂ Scale 1 (%) | ≤91 | 92-93 | 94-95 | ≥96 | | | |
| SpO₂ Scale 2 (%) | ≤83 | 84-85 | 86-87 | 88-92 ≥93 (Air) | 93-94 (02) | 95-96 (02) | ≥97 (O2) |
| Air or O₂ | | O2 | | Air | | | |
| Systolic Blood Pressure (mmHg) | ≤90 | 91-100 | 101-110 | 111-219 | | | ≥220 |
| Pulse (per minute) | ≤40 | | 41-50 | 51-90 | | | ≥131 |
| Consciousness | | | | Alert | | | CVPU |
| Temperature (°C) | ≤35 | | 35.1-36 | 36.1-38 | 38.1-39 | ≥39.1 | |

wherein "CVPU" stands for "Confusion but responds to Voice and Pain or Unresponsive." Accordingly, a hypercapnic patient (for which SpO₂ Scale 2 is utilized) with a respiration rate of 15 breaths per minute, an SpO₂ of 90%, a systolic blood pressure of 200 mmHg, a pulse of 60 beats per minute, a temperature of 37 °C, and who has not been provided supplemental oxygen and who is alert, may have a NEWS2 score of 0. In another example, a non-hypercapnic patient (for which SpO₂ Scale 2 is utilized) with a respiration rate of 15 breaths per minute, an SpO₂ of 97%, a systolic blood pressure of 200 mmHg, a pulse of 60 beats per minute, a temperature of 37 °C, and who has not been provided supplemental oxygen and who is alert, may have a NEWS2 score of 0. Notably, the NEWS2 score can range from 0 to 24.

Like a NEWS2 score, a MEWS score is a single numerical value that can be determined by summing additive factors together. The following Table 2 illustrates example additive factors corresponding to the MEWS scale.

**Table 2**

| **Additive Factor** | **3** | **2** | **1** | **0** | **1** | **2** | **3** |
|---|---|---|---|---|---|---|---|
| Respiration Rate (per minute) | ≥30 | 21-29 | 15-20 | 12-14 | 10-11 | 8-9 | ≤7 |
| Heart Rate (bpm) | ≥130 | 111-129 | 101-110 | 60-100 | 51-59 | 40-50 | ≤39 |
| O₂ Saturation (%) | | | | 95+ | 90-94 | 85-89 | <85 |
| Systolic Blood Pressure | ≥180 | 170-179 | 150-169 | 101-149 | 81-100 | 71-80 | <70 |
| Temperature (°C) | >39.6 | 38.6-39.5 | 37.8-38.5 | 36-37.7 | 35.1-35.9 | 34-35 | <34 |
| Pain | | | | No pain | Mild | Moderate | Severe |
| Neurological Status | Unresponsive | Reacting to pain! confused | Reacting to voice | Alert | | | |
| Urine Output | <20 ml/hr | ≤30 ml/hr | ≤50 ml/hr | 60 ml/hr | | | >30 ml/hr for 2 hrs |

The monitor 120 may include memory configured to at least temporarily store the first parameter(s) 122, the second parameter(s) 126, and/or any other parameters of the patient 102 received by the monitor 120. For example, the monitor 120 may receive multiple data packets including the first parameter(s) 122 and/or the second parameter(s) 126 at different times, output the most recently received of the first parameter(s) 122 and/or the second parameter(s) 126, and store previously received first parameter(s) 122 and/or the second parameter(s) 126. In some cases, the monitor 120 may further store parameters entered directly by the care provider 104 (e.g., level of pain, level of consciousness, etc.). In some examples, the monitor 120 may also store parameters derived at least partially based on the first parameter(s) 122 and/or the second parameter(s) 126, such as an EWS of the patient 102.

In various implementations, the monitor 120 may package the first parameter(s) 122 and/or the second parameter(s) 126 into EMR data 128 and transmit the EMR data 128 to an EMR system 130. The EMR system 130 may store an EMR of the patient 102. As used herein, the terms "electronic health record," "electronic medical record," "EMR," and their equivalents, may refer to a collection of stored data indicative of a medical history and/or at least one medical condition of an individual, wherein the stored data is accessible (e.g., can be modified and/or retrieved) by one or more computing devices. An EMR of an individual may include data indicating previous or current medical conditions, diagnostic tests, or treatments of the individual. For instance, the EMR may indicate demographics of the individual, parameters (e.g., vital signs) of the individual, notes from one or more medical appointments attended by the individual, medications prescribed or administered to the individual, therapies (e.g., surgeries, outpatient procedures, etc.) administered to the individual, results of diagnostic tests performed on the individual, identifying information (e.g., a name, birthdate, etc.) of the individual, or a combination thereof.

In various implementations, the EMR system 130 may use the EMR data 128 to update the EMR of the patient 102. For example, the EMR system 130 may update the EMR of the patient 102 to indicate a vital sign of the patient 102 included in the EMR data 128. In some cases, once the EMR data 128 is transmitted to the EMR system 130, the monitor 120 may delete the parameters stored in the memory of the monitor 120.

The EMR data 128 may be transmitted by the monitor 120 to the EMR system 130 in a single transmission. As used herein, the term "single transmission," and its equivalents, may refer to a single data flow, a single data stream, a single data packet, or a combination thereof. For example, the EMR data 128 may indicate multiple parameters among the first parameter(s) 122 and/or the second parameter(s) 126, even in cases where the first parameter(s) 122 and/or the second parameter(s) 126 are detected by multiple, independent devices. The EMR data 128 may indicate multiple parameters detected by multiple devices but may be transmitted to the EMR system 130 from a single device.

In some examples, the monitor 120 may receive sensor data including first parameter(s) 122, second parameter(s) 126, and/or any other sensor data in real-time. The monitor 120 may determine, based at least partly such sensor data, a baseline associated with the patient. For instance, the baseline may correspond to one or more of the vital sign(s) of the patient. The monitor 120 may determine the baseline based on one or more of the sensor data, historical data associated with the patient (e.g., such as by accessing patient information from the EMR and/or third party server(s) (not shown)), demographic data, and/or any other information available to the system 100 described herein. Accordingly, the baseline may be customized to the patient based on patient data (e.g., vital sign data, EMR data, demographic information, etc.). As described in greater detail below, the monitor 120 may generate a control chart associated with the patient. Generally, a control chart is a statistical process control tool used to view how a process changes over time. For instance, as described in greater detail below, a control chart comprises a central line associated with an average (e.g., mean), an upper line for the upper control limit, and a lower line for the lower control limit. These lines are traditionally determined using historical data. The control chart may comprise data associated with at least one vital sign over a period of time (e.g., 1 hour, 2 hours, or any other suitable time period). The control chart may further comprise an overlay that indicates the baseline customized to the patient. For instance, the overlay may comprise indications of a mean (e.g., average) associated with the particular vital sign and/or one or more indications of standard deviations that are customized to the patient based on one or more rules. In some examples, the monitor 120 determines the rule(s) to apply to the control chart based on data associated with the vital being monitored (e.g., industry standards, known characteristics, etc.) and/or patient health data. Accordingly, the monitor 120 may receive real-time continuous vitals for the patient for each time period (e.g., every 5 seconds, 10 seconds, and/or any other suitable time period) and can detect the deviations from an assumed distribution with every incoming new measurement. In some examples, the monitor 120 may perform actions including: (1) alerting a care provider of the deviations, (2) provide visual assistant of the alert, and/or (3) provide option(s) for accepting, changing, and/or removing a new baseline.

Accordingly, the monitor device 120 may provide a dynamic alarm setting based on real-time continuous multi-vitals measurements and provide customized control lines to enable a care provider to define more flexible and customized alarm rules that aims to capture more significant events and trends rather than traditional threshold mechanism. Accordingly, the customized control charts may reduce alarm fatigue while providing customized care for patients.

Although not illustrated in FIG. 1, in some implementations, an aggregator device may connect the monitor 120 to the EMR system 130. The aggregator, for example, may receive EMR data from multiple monitors including the monitor 120. The aggregator may generate aggregated data by combining the EMR data from the multiple monitors. The aggregator may transmit the aggregated data to the EMR system 130 in a single transmission. Thus, the aggregator may further limit the number of transmissions used to update the EMRs of various patients including the patient 102. In some cases, the aggregator is implemented in one or more servers on the premises of the clinical environment containing the monitor 120. In some implementations, the aggregator is implemented by one or more virtual machines (VMs) operating on servers in a remote, cloud-based environment.

In various cases, the monitor 120 may refrain from transmitting the EMR data 128 to the EMR system 130 until at least one confirmation process has been performed. The confirmation process(es), for example, are used to confirm whether the parameters (e.g., the first parameter(s) 122 and/or the second parameter(s) 126) are accurate. In some cases, even if the first parameter(s) 122 and/or the second parameter(s) 126 are continuously transmitted to the monitor 120, the monitor 120 may wait to report the first parameter(s) 122 and/or the second parameter(s) 126 to the EMR system 130 until they are confirmed. In some examples, if the first parameter(s) and/or the second parameter(s) 126 are not confirmed, the monitor 120 may refrain from sending the EMR data 128 to the EMR system 130 entirely. Thus, the monitor 120 may prevent the EMR system 130 from updating the EMR of the patient 102 with inaccurate information.

In the preferred embodiment of the invention, the monitor 120 determines that at least one of the first parameter 122 and the second parameter 126 is confirmed based on user input. In various implementations, the monitor 120 outputs the first parameter(s) 122 and/or the second parameter(s) 126 to the care provider 104, who can review the first parameter(s) 122 and/or the second parameter(s) 126. If the care provider 104 has reason to believe that the first parameter(s) 122 and/or the second parameter(s) 126 are accurate, the care provider 104 may confirm the first parameter(s) 122 and/or the second parameter(s) 126 with the monitor 120. For example, the input device of the monitor 120 may be configured to receive an input signal from the care provider 104 that confirms the first parameter(s) 122 and/or the second parameter(s) 126.

In some implementations, the monitor 120 determines that the first parameter(s) 122 and/or the second parameter(s) 126 are confirmed based on the first parameter(s) 122 and/or the second parameter(s) 126 themselves. In various implementations, a position or movement of the patient 102 may cause the vital sign sensor(s) 118 to inaccurately detect the first parameter(s) 122 of the patient 102. For example, movement of the patient 102 may move a finger-clip oximetry sensor attached to the patient 102, thereby introducing noise or artifact into an oximetry level detected by the oximetry sensor. In some examples, a blood pressure cuff may be configured to accurately detect the blood pressure of the patient 102 when the patient is sitting upright, and may inaccurately detect the blood pressure of the patient 102 when the patient is laying down. According to some examples, the patient 102 may leave the support structure 106 (e.g., for an appointment with another medical provider, a visit to a restroom, etc.), and the vital sign sensor(s) 118 may be unable to accurately detect the first parameter(s) 122 while the patient 102 is absent from the support structure 106. For example, the patient 102 may detach an impedance-based respiration sensor in order to leave the support structure 106, such that the impedance-based respiration sensor may be unable to detect the respiration rate of the patient 102.

The monitor 120 may determine the position or movement of the patient 102 based on the second parameter(s) 126. For example, the monitor 120 may determine that the patient 102 has moved based on a change in the weight detected by the load cell(s) 108, a change in the temperature detected by the temperature sensor(s) 122, image(s) detected by the camera(s) 114, audio detected by the microphone(s) 116, or any combination thereof. For instance, the monitor 120 may recognize the patient 102 in images detected by the camera(s) 114 using image recognition techniques (e.g., facial recognition) and identify movement of the patient 102 based on differences between the images. In some examples, the monitor 120 may determine whether the patient 102 is sitting in an upright position or is laying down on the support structure 106 based on the second parameter(s) 126 detected by the various sensors integrated with the support structure 106. In some cases, the monitor may identify a movement of a limb (e.g., an arm or a leg) of the patient 102.

The monitor 120 may confirm the first parameter(s) 122 and/or the second parameter(s) 126 based on the movement or position of the patient 102. For instance, the monitor 120 may confirm the first parameter(s) 122 and/or the second parameter(s) 126 detected during a time interval based on determining that the movement (e.g., a velocity, acceleration, jerk, etc.) of the patient 102, during the time interval, is less than a threshold movement (e.g., a threshold velocity, acceleration, jerk, etc.). In some cases, the monitor 120 may confirm the first parameter(s) 122 and/or the second parameter(s) 126 based on determining that the position of the patient 102 is substantially unchanged during the time interval. For example, the monitor 120 may determine that the patient 102 has remained sitting upright during the time interval or has remained laying down during the interval. The movement or position of the patient 102 may therefore be an independent factor for confirming the first parameter(s) 122 and/or the second parameter(s) 126.

In some cases, the monitor 120 may determine that the first parameter(s) 122 and/or the second parameter(s) 126 are confirmed based on a comparison with a physiological range. For example, if the first parameter(s) 122 indicate a heart rate of 0, the monitor 120 can conclude that the heart rate is inaccurate and may refrain from confirming the heart rate. In some examples, the monitor 120 may compare the first parameter(s) 122 and/or the second parameter(s) 126 to one or more ranges indicating physiological ranges. If the monitor 120 determines that the first parameter(s) 122 and/or the second parameter(s) 126 are within the physiological ranges, the monitor 120 may transmit the EMR data 128 indicating the first parameter(s) 122 and/or the second parameter(s) 126 to the EMR system 130.

The monitor 120 provides a number of advantages over existing solutions. First, by aggregating the first parameter(s) 122 and/or the second parameter(s) 126 into the EMR data 128, the monitor 120 may transmit multiple parameters of the patient 102 to the EMR system 130 over a single communications interface, data packet, and/or data flow. This provides an improvement over an alternative system in which the individual vital sign sensor(s) 118 could transmit the first parameter(s) 122 directly to the EMR system 130 (over a first transmission) and the transmitter 124 of the support structure 106 could transmit the second parameter(s) 126 directly to the EMR system 130 (over a second transmission). In examples in which an organization controlling the EMR system 130 charges the clinical environment by communication to the EMR system 130, the monitor 120 enables the clinical environment to conserve money as compared to the alternative system.

Second, the monitor 120 enables the care provider 104 to efficiently update the EMR of the patient 102 with minimal intervention. In the alternative system, the care provider 104 may manually write down the first parameter(s) detected by the vital sign sensor(s) 118, before updating the EMR of the patient 102 by entering the parameter(s) into a separate computing device. By aggregating and outputting multiple parameters together, in some examples, the monitor 120 may reduce the number of manual steps that the care provider 104 performs in order to update the EMR of the patient 102.

Third, by confirming the first parameter(s) 122 and/or the second parameter(s) 126 before the EMR data 128 is transmitted to the EMR system 130, the monitor 120 may improve the accuracy of the EMR of the patient 102 with minimal input from the care provider 104. For example, in an instance of the alternative system in which the vital sign sensor(s) 118 and support structure 106 automatically transmit the first parameter(s) 122 and second parameter(s) 126 to the EMR system 130 without independent confirmation, it may be possible for the EMR system 130 to update the EMR of the patient 102 based on erroneous parameters. In contrast, the monitor 120 may transmit the EMR data 128 to the EMR system 130 in response to confirming that the first parameter(s) 122 and/or second parameter(s) 126 are accurate.

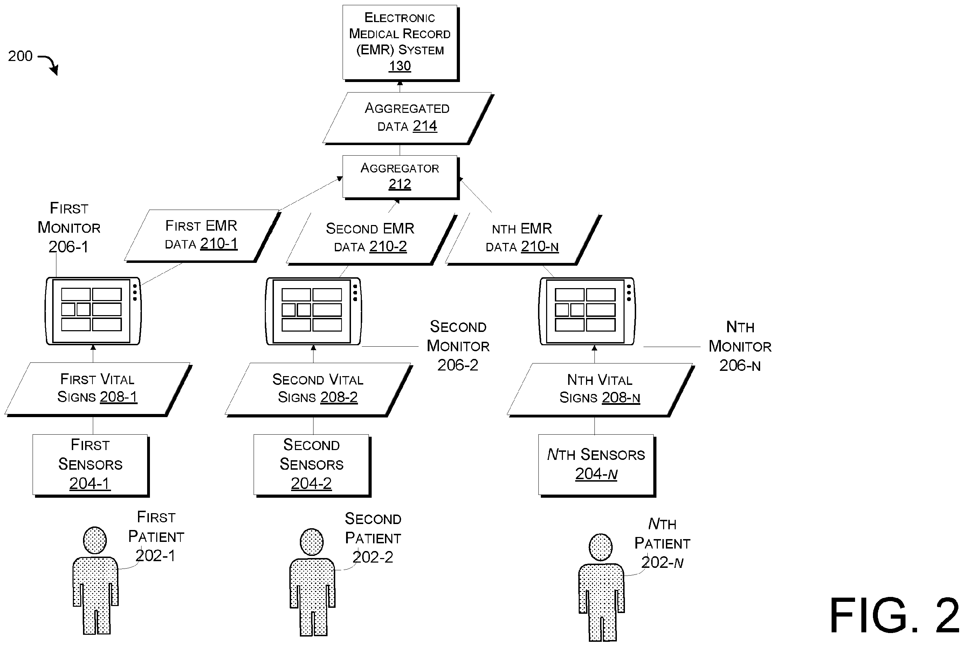

FIG. 2 illustrates an example environment 200 wherein parameters from multiple patients are reported to the EMR system 130. The environment 200 includes first through nth patients 202-1 to 202-*n*, wherein *n* is a positive integer. For example, one of the first through *n*th patients 202-1 to 202-*n* may be the patient 102 described above with reference to FIG. 1. The first through *n*th patients 202-1 to 202-*n* are respectively monitored by first through *n*th sensors 204-1 to 204-*n*. In some cases, the first through *n*th sensors 204-1 to 204-*n* include the sensors integrated into the support structure 106 and/or the vital sign sensor(s) 118 described above with respect to FIG. 1.

The first through *n*th patients 202-1 to 202-*n* may be respectively associated with first through *n*th monitors 206-1 to 206-*n*. As illustrated in FIG. 2, each of the first to *n*th patients 202-1 to 202-*n* may be associated with an independent one of the first through *n*th monitors 206-1 to 206-*n*. However, in some implementations of the present disclosure, a single one of the first through nth monitors 206-1 to 206-*n* may be associated with multiple patients among the first through *n*th patients 202-1 to 202-*n*. For example, a care provider may move a single one of the monitors 206-1 to 206-*n* between rooms of multiple patients among the first through *n*th patients 202-1 to 202-*n*.

The first to nth sensors 204-1 to 204-*n* may transmit first to *n*th vital signs 208-1 to 208-*n* to the first to *n*th monitors 206-1 to 206-*n*. In various cases, each of the first to *n*th sensors 204-1 to 204-*n* may transmit multiple vital signs to the corresponding one of the first to *n*th monitors 206-*n*. For example, the first vital signs 208-1 may include at least two of a respiratory rate, an oxygen saturation, a blood pressure, a heart rate, and a level of consciousness of the first patient 202-1. Each of the first to nth monitors 206-1 to 206-*n* may at least temporarily store and combine the vital signs among the first to *n*th vital signs 208-1 to 208-*n* it receives. For example, the first monitor 206-1 may at least temporarily store the first vital signs 208-1.

In various implementations, the first to *n*th monitors 206-1 to 206-*n* may determine whether the first to *n*th vital signs 208-1 to 208-*n* are confirmed. For instance, the second monitor 206-2 may determine whether parameters from a support structure configured to support the second patient 202-2 confirm the accuracy of the second vital signs 208-2, whether a user input from a care provider confirms the accuracy of the second vital signs 208-2, whether the second vital signs 208-2 are within physiological ranges, or a combination thereof. In various implementations, the first to nth monitors 206-1 to 206-*n* may discard any of the first to *n*th vital signs 208-1 to 208-*n* that are not confirmed. For instance, if the second monitor 206-2 is unable to confirm the accuracy of the second vital signs 208-2, the second monitor 206-2 may delete the second vital signs 208-2 without reporting them.

If the first to *n*th vital signs 208-1 to 208-*n* are confirmed, the first to *n*th monitors 206-1 to 206-*n* may transmit first to *n*th EMR data 210-1 to 210-*n* to an aggregator 212. The aggregator 212 may at least temporarily store the first to *n*th EMR data 210-1 to 210*-n.* In addition, the aggregator 212 may transmit aggregated data 214 to the EMR system 130. The aggregated data 214 may include the first to *n*th EMR data 210-1 to 210*-n.* In various implementations, the aggregated data 214 may be transmitted to the EMR system 130 in a single transmission (e.g., a unidirectional data flow). Thus, the aggregator 212 may further reduce the number of transmissions used to report the first to *n*th vital signs 208-1 to 208-*n* from the first to *n*th patients 202-1 to 202-*n* to the EMR system 130.

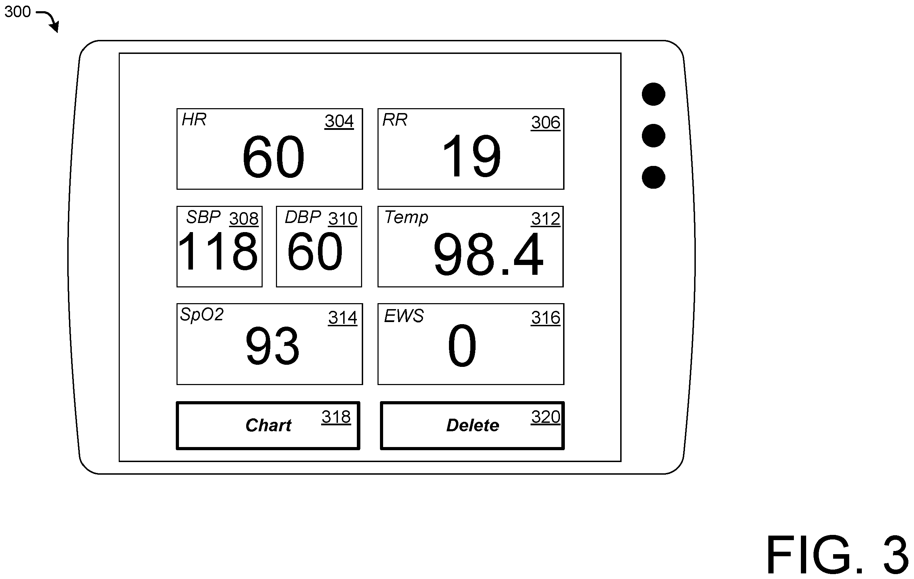

FIG. 3 illustrates an example of a monitor 300 used to aggregate, display, and report multiple parameters of a patient. As illustrated in FIG. 3, the monitor 300 includes a display configured to visually output information. In particular, the display outputs various user interface elements that graphically represent different vital signs, such as heart rate 304, respiration rate 306, systolic blood pressure 308, diastolic blood pressure 310, temperature 312, and oxygenation 314 (e.g., pulse oxygenation) of the patient. Further, the display may output other types of parameters, such as an early warning score (EWS) 316 of the patient.

In various implementations, the monitor 300 may update the user interface elements in real-time as the vital signs are detected from the patient. For example, the monitor 300 may receive, from a heart rate sensor, data indicative of the heart rate of the patient at a first time. The monitor 300 may initially display the heart rate at the first time as the heart rate 304. The monitor 300 may subsequently receive, from the heart rate sensor, data indicative of the heart rate of the patient at a second time. The monitor 300 may update the heart rate 304 output by the display based on the heart rate of the patient at the second time. In various implementations, the monitor 300 may store parameters that are not currently output by the display. For instance, the monitor 300 may store the heart rate of the patient at the first time even though the display may be outputting the heart rate of the patient at the second time as the heart rate 304.

The monitor 300 may further include a selector 318 (illustrated as "Chart 318"). In various implementations, the selector 318 may include any sort of input device configured to receive an input signal of a user, such as a care provider. In the example of FIG. 3, the display may be a touchscreen and the selector 318 may be visually output by the display over a region corresponding to one or more touch sensors. The user may provide an input signal to the monitor 300 by touching the touch sensor(s) overlapping the region of the selector 318 output by the display.

In various implementations, the user may view the parameters output by the monitor 300 and provide the input signal in response to determining that the parameters appear to be accurate. For example, the user may view the patient and the sensors first-hand and determine whether the parameters appear to match the condition of the patient. Upon confirming that the parameters are accurate, the user may provide the input signal to the selector 318 (e.g., touch the selector 318). Based on the input signal, the monitor 300 may transmit the multiple parameters displayed by the monitor 300 to an EMR system in a single data packet, data stream, and/or data flow.

The monitor 300 may also include a discard element 320. In various implementations, the discard element 320 may include any sort of input device configured to receive an input signal of a user, such as a care provider. In the example of FIG. 3, the display may be a touchscreen and the discard element 320 may be visually output by the display over a region corresponding to one or more touch sensors. The user may provide an input signal to the monitor 300 by touching the touch sensor(s) overlapping the region of the discard element 320 output by the display.

According to some implementations, the user may prevent the monitor 300 from transmitting the parameters displayed by the monitor 300 to the EMR system by providing an input signal to the discard element 320. In a particular example in which the display outputs a heart rate 304 of 0, but the patient is responsive and conscious, the user may determine that the heart rate 304 is inaccurate and may refrain from confirming the accuracy of the parameters. In some cases, the user may provide an input signal to the discard element 320. In various implementations, the monitor 300 may delete the inaccurate parameters based on the discard element 320 receiving the input signal.

A control chart may be displayed on the display of the monitor 300 described in FIG. 3. In some examples, the user interface may be displayed in addition to or in place of one or more of the elements described above in FIG. 3.

The control chart may include one or more data points. The data points may correspond to at least one vital sign of a patient that is being monitored for a period of time. In this example, the vital sign is represented as heart rate. As noted above, the monitor 120 and/or processor(s) of the monitor 120 may utilize one or more rules to determine an initial baseline of a patient at check in. For instance, the rules may be based on one or more of Westgard rules, Western electric rules, Nelson rules, and/or customized rules input by a care provider. In some examples, the rule(s) are identified using a pre-trained model and/or pre-trained weighted model stored in memory. In some examples, the pre-trained model is pre-trained using machine learning techniques. In some examples, the monitoring device 120 stores machine-trained data models for use during operation. Machine learning techniques include, but are not limited to supervised learning algorithms (e.g., artificial neural networks, Bayesian statistics, support vector machines, decision trees, classifiers, k-nearest neighbor, etc.), unsupervised learning algorithms (e.g., artificial neural networks, association rule learning, hierarchical clustering, cluster analysis, etc.), semi-supervised learning algorithms, deep learning algorithms, etc.), statistical models, etc. In some examples, the one or more rule(s) represent a threshold (e.g., a deviation from the baseline (e.g., represented as the mean)), that, when exceeded, the monitor 120 generates an alert for the care provider 104.

In some examples, the monitor determines an initial baseline associated with a patient at check-in. The initial baseline may be based on data associated with the patient and/or data associated with being monitored. For instance, an initial baseline may be determined based on mean averages and/or standard deviations associated with one or more of age, gender, demographics, known diagnosis, and/or any other relevant information. In some examples, monitor stores the mean averages and/or standard deviations associated with one or more of age, gender, diagnosis, vital sign, etc. in memory. In other examples, the monitor accesses the mean average(s) and/or standard deviation(s) from an external server (e.g., such as the EMR, or other server (not shown)).

In some examples, the initial baseline may be determined based on monitoring the patient over a period of time. In some examples, the rules may specify one or more basic alarm limits, such as setting the alarm limits to desired standard deviations based on default demographics associated with the patient and/or historical data associated with the patient. In some examples, such as where there is no historical data associated with the patient, the rules may indicate that the alarm limits are set based on industry standards. In some examples, the monitoring device 120 may adjust the initial baseline after a period of time (e.g., every 10 hours, 12 hours, or any other suitable period of time). For instance, the monitoring device 120 may determine, based on sensor data over a period of time that the average heart rate of the patient is higher or lower than the initial baseline. The monitoring device 120 may output an indication to the display (not shown) to alert a care provider of the suggested change. In other examples, the monitoring device 120 may automatically adjust the baseline based on real-time sensor data and without input from the care provider 104.

The display may comprise one or more selectable elements. For instance, a first selectable element may be selectable to enable a care provider to approve the baseline suggested by the control chart. In some examples, the care provider may select, such as via a touch input to the display, the first selectable element in order to approve the control chart and/or standard deviations.

In some examples the care provider may select a second selectable element in order to change one or more of the standard deviations, mean, and/or severity level associated with an alert. For instance, a rule associated with the control chart may comprise generating an alert when the patient vital passes a first threshold (e.g., such as a first standard deviation), where the alert is associated with a lower level of severity. The rules may further specify generating alert(s) indicating higher level(s) of severity when the vital sign passes one or more higher thresholds (e.g., a second standard deviation and/or a third standard deviation). By selecting the second selectable element, a care provider may customize and/or adjust the rule(s), such as removing alert(s) associated with one or more of the standard deviation(s), adjusting severity level(s) associated with the alert(s), adding alert(s), adding severity level(s), changing the mean, changing one or more of the standard deviation(s), removing outlier data, etc.

In some examples, selecting the second selectable element enables a care provider to increase and/or decrease the baseline. For instance, the care provider may provide input (e.g., such as dragging/dropping, swiping up, or any other suitable type of input) to increase the baseline. In this example, the control chart may update to provide visual indicia (e.g., change in color of one or more sensor data, highlighting, change in shape, size, etc. of sensor data, or any other suitable type of visual indicia) of the adjustment and/or one or more sensor data impacted by the adjustment. For instance, the visual indicia may be highlighting an area above the third standard deviation to indicate a new area and/or sensor data that would result in an alarm based on the new baseline. In some examples, the care provider can save the new baseline and/or alarm setting in memory of the monitor.

In some examples, the display includes a third selectable element. A care provider 104 may provide input selecting the third selectable element in order to remove outlier data, reject the suggested control lines, reject suggested alarm settings, etc.

Accordingly, the monitor device 120 may provide a dynamic alarm setting based on real-time continuous multi-vitals measurements and provide customized control lines to enable a care provider to define more flexible and customized alarm rules that captures more significant events and trends, thereby reducing alarm fatigue. Accordingly, the customized control charts may reduce alarm fatigue while providing customized care for patients.

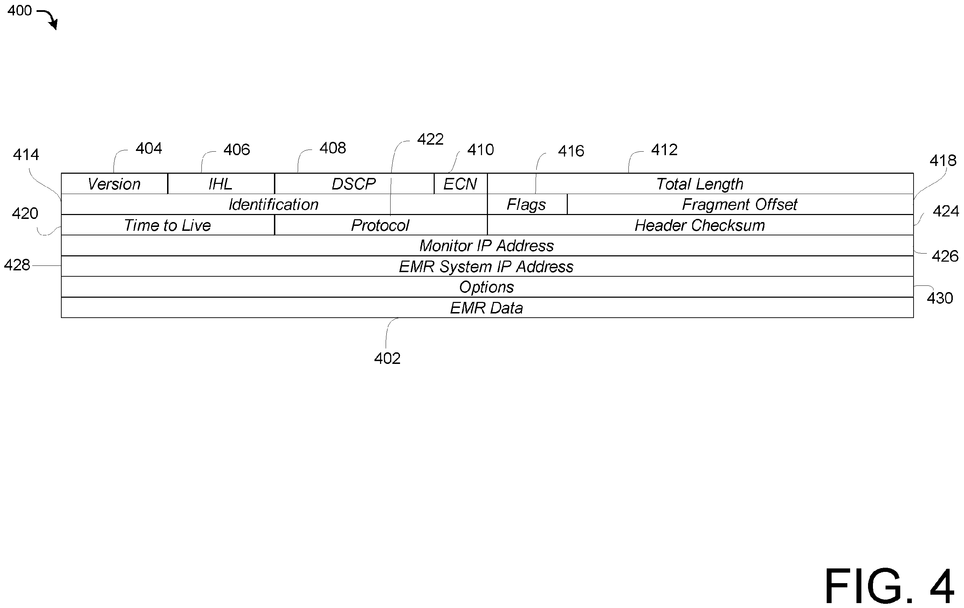

FIG. 4 illustrates an example data packet 400 carrying EMR data 402. The data packet 400 is an IP version 4 (IPv4) data packet, but implementations are not so limited. For example, IP version 6 (IPv6) data packets and/or BLLTETOOTH transmissions can be used to carry the EMR data 402, in some cases. As illustrated, the data packet 400 includes multiple fields, such as a version field 404, an Internet Header Length (IHL) field 406, a Differentiated Services Code Point (DSCP) field 408, an Explicit Congestion Notification (ECN) field 410, a total length field 412, an identification field 414, a flags field 416, a fragment offset field 418, a time to live (TTL) field 420, a protocol field 422, a header checksum field 424, a monitor IP address field 426, an EMR system IP address field 428, and an options field 430. The options field 236 is an optional field.

The version field 404 may be a four-bit field. In the data packet 400, the version field 210 may be equal to 4, representing an IPv4 packet.

The IHL field 406 may be a four-bit field. The IHL field 406 may specify the length of a header of the data packet 400. A minimum value of the IHL field 406 may be equal to five. When the IHL field 406 is equal to five, it may indicate that the length of the header of the data packet 400 is 160 bits or 20 bytes.

The DSCP field 408 may indicate a Type of Service (ToS) of the data packet 400. For instance, the DSCP field 408 may specify a differentiated service (DiffServ).

The ECN field 410 may provide a notification of end-to-end network congestion. In various implementations, the ECN field 410 is optional, and may be omitted from the data packet 400.

The total length field 412 may be a 16-bit field that defines a size of the entire data packet 400. A minimum size of the data packet 400 that is identified in the total length field 412 may include a length of both the IPv4 header and a UDP header in the data packet 400.

The identification field 414 may be used to identify a group of fragments of an IP datagram. The flags field 416 may be a three-bit field that is used to control or identify the fragments. A first bit of the flags field 416 may be reserved and equal to zero, a second bit may indicate that fragmentation can be used to route the data packet 400, and the third bit indicates that more fragments can be used to route the data packet 400. In various implementations, the flags field 416 may indicate that the data packet 400 is an unfragmented packet. The fragment offset field 418 may be a 13-bit field that specifies an offset of a particular fragment relative to a beginning of an original unfragmented IP datagram.

The TTL field 420 may be an eight-bit field that specifies a value of a maximum number of hops (or a maximum number of times) that the data packet 400 can continue being routed through a communications network. In particular implementations, the TTL field 420 may be set to a predetermined value. The TTL field 420 may be set to a maximum value of 255.

The protocol field 422 may define the protocol used in the rest of the data packet 400. The checksum field 424 may be a 16-bit field that can be used to check of errors in the header as it is transferred through the network. When the data packet 400 is received by a particular network node, the network node may calculate a checksum for the header and compare the calculated checksum to the checksum field 424. If the values are mismatched, the particular node discards the data packet 400. If the values are matched, the particular node may process and/or forward the data packet 400.

The monitor IP address 426 may specify an IP address identifying the monitor transmitting the data packet 400. For example, if the data packet 400 is generated by the monitor 120 described above with reference to FIG. 1, then the monitor IP address 426 may reflect the IP address of the monitor 120. The EMR system IP address 428 may specify an IP address of the EMR system that is the destination of the data packet 400.

The options field 430 may, in some cases, be omitted from the header of the data packet 400. In various examples, the options field 430 may be substituted for necessary padding to ensure that the header of the data packet 400 contains an integer number of 32-bit words.

The header of the data packet 400 may include the version 404, the IHL 406, the DSCP 408, the ECN 410, the total length 412, the identification 414, the flags 416, the fragment offset 418, the TTL 420, the protocol 422, the header checksum 424, the monitor IP address 426, the EMR system IP address 428, and the options 430. A payload of the data packet 400 may include the EMR data 402. The EMR data 402 may indicate one or more parameters of a patient. For example, the EMR data 402 may indicate one or more vital signs of the patient. In some examples, the single data packet 400 can indicate multiple parameters (e.g., vital signs) of the patient that were detected using multiple, independent sensor devices. In various implementations, the EMR data 402 may further indicate an identity of the patient. For example, the EMR of the patient may be associated with a particular identifier that is indicated in the EMR data 402. Accordingly, the EMR system may be able to identify which EMR, among multiple EMRs stored by the EMR system, belongs to the patient whose parameter(s) are indicated in the EMR data 402.

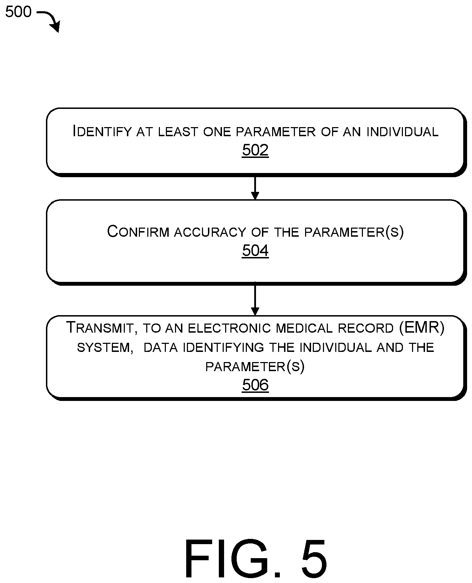

FIG. 5 illustrates an example process 500 for updating an EMR of an individual based on parameters of the individual. The process 500 may be performed by an entity including at least one of the monitor 120, the first monitor 206-1, the second monitor 206-2, the nth monitor 206-n, the aggregator 212, or the monitor 300 described above with reference to FIGS. 1-3. In some examples, the entity is a processor executing instructions stored in memory.

At 502, the entity identifies at least one parameter of the individual. In some examples, the parameter(s) include one or more vital signs. The parameter(s), for example, may be detected by one or more sensors. In some cases, the parameter(s) are at least partially detected by a sensor integrated into a support structure configured to support the individual. For instance, the support structure may be a hospital bed.

In various implementations, the entity receives the parameter(s) from the sensor(s). The parameter(s) may be transmitted to the entity over one or more communications networks. In some examples, the entity outputs the parameter(s) to a user. For instance, the entity may include a display configured to visually display the parameter(s). In some examples, the entity at least temporarily stores the parameter(s).

At 504, the entity confirms the accuracy of the parameter(s). In some examples, the entity confirms the accuracy of the parameter(s) by determining that the parameter(s) are within one or more physiological ranges. In some cases, the entity confirms the accuracy of the parameter(s) based on a position of the individual during the time interval at which the parameter(s) were detected. For example, the entity may identify one or more other parameters indicative of the position of the individual, and determine based on the other parameter(s) that the position of the individual was substantially unchanged during the time interval. In some implementations, the entity confirms the accuracy of the parameter(s) based on an input signal received from a user. For instance, the user may view the parameter(s) output by the entity and may subsequently provide the input signal to the entity.

At 506, the entity transmits, to an EMR system, data identifying the individual and the parameter(s). In various implementations, the data may be included in a single data packet, data stream, and/or data flow. Thus, in cases where the entity identifies and confirms multiple parameters, the multiple parameters are aggregated into the data provided to the EMR system. Upon receiving the data from the entity, the EMR system may update the EMR of the individual. In some cases, the entity may delete the stored parameter(s) upon transmitting the data to the EMR system.

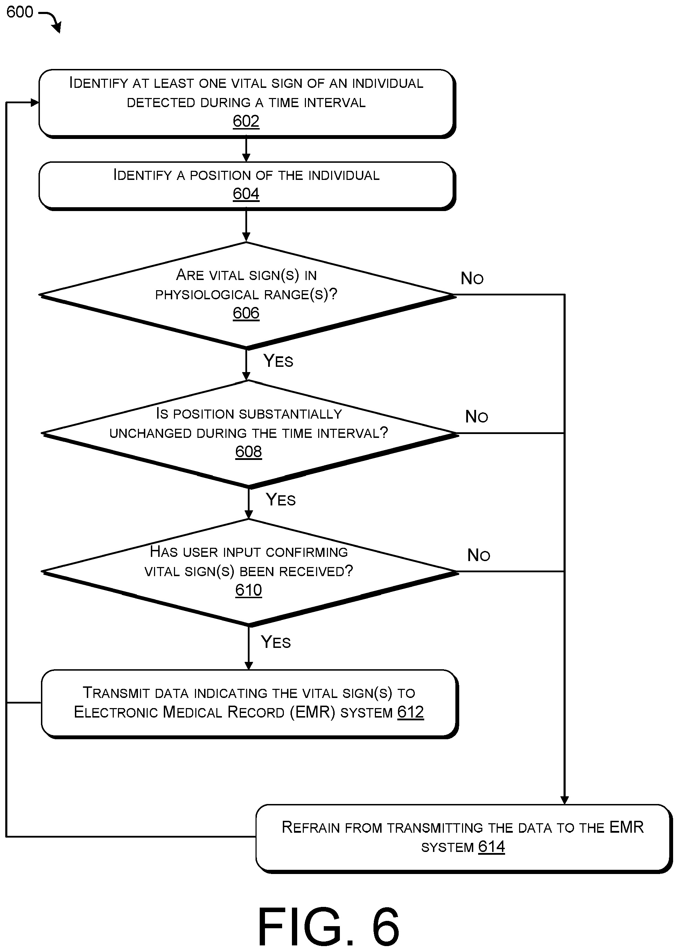

FIG. 6 illustrates an example process 600 for selectively reporting at least one vital sign of an individual to an EMR system. The process 600 may be performed by an entity including at least one of the monitor 120, the first monitor 206-1, the second monitor 206-2, the nth monitor 206-n, the aggregator 212, or the monitor 300 described above with reference to FIGS. 1 to 3. In some examples, the entity includes a processor configured to execute instructions stored by memory.

At 602, the entity identifies the vital sign(s) of the individual. The vital sign(s) are detected during a time interval. In some cases, the entity includes one or more sensors that detect at least a portion of the vital sign(s) directly. At least one of the sensor(s) may be integrated into a support structure (e.g., a hospital bed) supporting the individual. In some examples, the entity receives one or more signals indicative of at least a portion of the vital sign(s) from the sensor(s). According to some implementations, the entity receives, from a user, one or more input signals indicative of at least a portion of the vital sign(s). In some cases, the entity derives the vital sign(s) based on signals received from the sensor(s). In various implementations, the entity may at least temporarily store the vital sign(s).

At 604, the entity identifies a position of the individual. In various implementations, the entity may identify the position of the individual based on data captured by the support structure supporting the individual. For example, the support structure may include one or more sensors (e.g., load cells, temperature sensors, microphones, cameras, etc.) configured to generate data that is indicative of the position and/or movement of the individual. The entity may derive the position and/or movement of the individual based on the data from the sensor(s) integrated into the support structure.

At 606, the entity determines whether the vital sign(s) are within one or more physiological ranges. The physiological range(s) for example, may be indicative that the individual is alive. A physiological heartbeat (or pulse rate) range may be 30 to 120 beats per minute (BPM), wherein the physiological range includes tachycardic and bradycardic heart rates. For example, a heart rate of 0 bpm may be outside of the physiological heartbeat range and more likely to be indicative of a problem with the sensor detecting heart rate than an accurate reading of the heart rate of the individual. A physiological respiratory rate range may be 10 to 30 breaths per minute. For instance, a respiratory rate of 0 breaths per minute may be outside of the physiological respiratory rate range and more likely to be indicative of a problem with the sensor detecting the respiratory rate than an accurate reading of the respiratory rate of the individual. A physiological body temperature range may be 95 to 110 degrees Fahrenheit. For example, a body temperature of less than 80 degrees maybe outside of the physiological body temperature range and more likely to be indicative of a problem with the temperature sensor than an accurate reading of the temperature of the individual. A physiological systolic blood pressure range may be 80 to 150 and a physiological diastolic blood pressure range may be 40 to 110. For example, a detected systolic blood pressure of 0 may be more likely to be indicative of an erroneous reading by the blood pressure sensor than an accurate reading of the blood pressure of the individual.

If the entity determines that the vital sign(s) are within the physiological range(s) at 606, then the process 600 proceeds to 608. At 608, the entity determines whether the position of the individual is substantially unchanged during the time interval. For example, the position of the individual may be substantially unchanged if the individual has remained laying down, or has remained sitting upright, throughout the time interval. In some cases, the position of the individual may be substantially unchanged if the individual has remained supported by the support structure throughout the time interval (e.g., the individual did not leave the support structure during the time interval). In some examples, the position of the individual may be substantially unchanged if the individual has not rolled over or bent or straightened their limbs (e.g., legs and/or arms) during the time interval. In some cases, the position of the individual may be substantially unchanged if a movement (e.g., a velocity, an acceleration, a jerk, etc.) of at least a portion of the individual (e.g., an arm) is less than a threshold level during the time interval.

If the entity determines that the position of the individual is substantially unchanged during the time interval at 608, then the process 600 proceeds to 610. At 610, the entity determines whether a user input confirming the vital sign(s) has been received. In various implementations, the entity may output the vital sign(s) to a user, such as a care provider. The user may view the vital sign(s) holistically at the bedside of the individual, in some cases. In some examples, the user may provide the user input to the entity upon determining that the vital sign(s) appear accurate. The entity may include an input device configured to receive the user input from the user.

If the entity determines that the user input confirming the vital sign(s) has been received at 610, then the process 600 proceeds to 612. At 612, the entity transmits data indicating the vital sign(s) to an EMR system. In some cases, the entity may transmit the data to an aggregator that transmits the data to the EMR system. In various implementations, the entity or the aggregator may transmit the data indicating the vital sign(s) to the EMR system in a single transmission. For example, the data may be a single data packet transmitted to the EMR system. In various implementations, the data may be a single transmission even if the data indicates multiple vital signs, captured by multiple sensors. In some implementations, the entity may delete the vital sign(s) stored by the entity after the data indicating the vital sign(s) is transmitted to the EMR system.

If, on the other hand, the entity determines that the vital sign(s) are outside of the physiological range(s) at 606, that the position of the patient is substantially changed during the time interval at 608, or that the user input confirming the vital sign(s) has not been received at 610, then the process 600 proceeds to 614. At 614, the entity refrains from transmitting the data to the EMR system. In some implementations, the entity may delete the vital sign(s) stored by the entity. After 612 or 614, the process 600 returns to 602.

An example process for generating customized control charts is described below. The example method is a logical flow graph, each operation of which represents a sequence of operations that may be implemented in hardware, software, or a combination thereof. In the context of software, the operations represent computer-executable instructions stored on one or more computer-readable storage media that, when executed by one or more processors, perform the recited operations. Generally, computer-executable instructions include routines, programs, objects, components, data structures, and the like that perform particular functions or implement particular abstract data types. The order in which the operations are described is not intended to be construed as a limitation, and any number of the described operations may be combined in any order and/or in parallel to implement the processes. Although any of the processes or other features described with respect to the method may be performed by processor(s) and/or monitoring device 120, for ease of description, the example method will be described below as being performed by processor(s) of the monitoring device 120 unless otherwise noted.

In step 1, the processor(s) receive an input indicative of a request or an instruction to perform continuous monitoring of a patient. For instance, in some examples, the input is received when a patient is checked in to a health care facility. In other examples, the indication is received for a patient that was previously checked in (e.g., previously admitted and/or already receiving treatment). In some examples, the indication is received from a care provider. The indication may include one or more vital sign(s) of the patient to be continuously monitored, a time period for the vital sign(s) to be monitored, and/or patient data (e.g., age, gender, demographic, known condition, etc.).

In step 2, the processor(s) determines a baseline associated with the patient. In some examples, the baseline may be determined based on the patient data. Additionally, or alternatively, the processor(s) may determine the baseline using real-time data corresponding to one or more of the vital sign(s) included in the indication. In some examples, the baseline represents the average value of the vital sign being monitored. For instance, where the vital sign corresponds to heart rate, the baseline may represent a mean of the recorded heart rate values of the patient and/or a mean of previously recorded values.

In step 3, the processor(s) may identify one or more rule(s). For instance, the rule(s) may be stored in memory of the monitoring device 120. In some examples, the rule(s) may be pre-set. The rule(s) may comprise one or more of Westgard rules, Western electric rules, Nelson rules, and/or customized rules set by a care provider. In some examples, the processor(s) may determine one or more deviation(s) from the baseline based at least in part on the rule(s) and/or associated information (e.g., demographics, age, gender, diagnosis) of the patient. For instance, where heart rate of the patient is being continuously monitored, the processor(s) may identify rule(s) that specify to generate an alarm with a low alert level where a single heart rate outside of three standard deviations from the baseline is detected and/or to generate an alarm with a medium alert level where two heart rates outside of two standard deviations from the baseline are detected within a particular period of time (1 minute, 5 minutes, 30 minutes, or any other suitable period of time). In some examples, the rule(s) may indicate when to notify a care provider of one or more trend(s) (upward, downward, etc.) associated with the real-time data.

In some examples, the processor(s) may identify the rule(s) using a pre-trained model and/or pre-trained weighted model stored in memory. In some examples, the pre-trained model is pre-trained using machine learning techniques. In some examples, the monitoring device 120 stores machine-trained data models for use during operation. Machine learning techniques include, but are not limited to supervised learning algorithms (e.g., artificial neural networks, Bayesian statistics, support vector machines, decision trees, classifiers, k-nearest neighbor, etc.), unsupervised learning algorithms (e.g., artificial neural networks, association rule learning, hierarchical clustering, cluster analysis, etc.), semi-supervised learning algorithms, deep learning algorithms, etc.), statistical models, etc.

In some examples, the rules and/or parameters associated with the rules may be preset (e.g., default rules). For instance, where heart rate is being continuously monitored, the default rules and/or default parameters may comprise: alert for high heart rate where (i) vitals: heart rate; (ii) control line: 2 standard deviation; (iii) trend: upward; (iv) number of samples: at least two consecutive samples; (v) severity of the alert: high. Default rules and/or default parameters for alerting a care provider of a low heart rate may comprise: (i) vitals: heart rate; (ii) control line: 3 standard deviations; (iii) trend: downward; (iv) number of samples: at least 3 consecutive samples; (v) severity of the alert: high. Default rules and/or default parameters for alerting a care provider of an upward trend alarm may comprise: (i) vitals: heart rate; (ii) control line: previous measurement; (iii) trend: upward; (iv) number of samples: at least 7 consecutive samples; (v) severity of the alert: medium. In some examples, the care provider may adjust one or more of the rules and/or parameters. The care provider may additionally and/or alternatively adjust alarm settings and/or alarm states (e.g., high, medium, low).

In some examples, the rules and/or parameters for the rules may be associated with multiple vitals being measured. In this example, alarms may be triggered where parameters for multiple vitals are met. For instance, default rules and/or default parameters for alerting a care provider of an alarm associated with multiple vitals may comprise: (i) vitals: [heart rate, respiration rate]; (ii) control line: 2 standard deviations; (iii) direction: [any, any]; (iv) number of samples: at least 7 consecutive samples; (v) severity of the alert: high.

In step 4, the processor(s) may generate the control chart. In some examples, the control chart is generated based at least in part on the real-time data and the rule(s). In some examples, the control chart comprises an overlay associated with patient vitals (e.g., real-time data). In some examples, an initial baseline associated with the patient is adjusted based on the real-time data indicated by the overlay. For instance, the initial baseline may be based at least in part on historical data associated with the patient and/or one or more similar patient(s) (e.g., identified based on age, gender, demographic information, etc.). After collecting the real-time data, the overlay may include an indication to update the initial baseline based on the real-time data. For instance, where the real-time data indicates the patient's heart rate baseline is higher than the initial baseline, the overlay may include a suggestion to update the initial baseline. In some examples, the processor(s) may automatically and dynamically update the initial baseline based on the real-time data.

In step 5, the processor(s) may output the control chart for display. For instance, the control chart may be displayed to a care provider. In some examples, the care provider may determine whether to adjust the initial baseline and/or any rules/parameters associated with the one or more vital sign(s) being monitored.

In step 6, the processor(s) determines whether one or more trigger(s) have been detected. For instance, the trigger(s) may correspond to one or more of the rule(s) and may identify when an initial baseline associated with a patient should be updated. For instance, after outputting the control chart for display, the processor(s) may continue to receive and monitor sensor data, real-time data, and/or other data associated with a patient. In some examples, the mean (e.g., average) of the vital sign being measured may increase or decrease over time. In this example, the processor(s) may detect a trigger associated with adjusting the initial baseline based on determining that the mean associated with the vital has increased and/or decreased more than a threshold amount (e.g., 5%, 10%, or any other suitable threshold) over a period of time (e.g., seconds, minutes, hours, etc.). In some examples, the threshold amount(s) may be preset and stored in memory of the monitor. In other examples, the threshold amount(s) may be customized by a care provider.

Where the processor(s) do not detect a trigger, the method returns to step 5 and the control chart is displayed to a care provider. Where the processor(s) detect one or more trigger(s), the method returns to step 2, where an updated baseline is dynamically determined and/or adjusted.

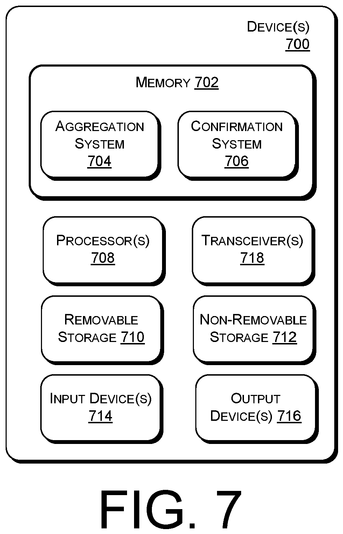

FIG. 7 illustrates at least one example device 700 configured to enable and/or perform the some or all of the functionality discussed herein. Further, the device(s) 700 can be implemented as one or more server computers, a network element on a dedicated hardware, as a software instance running on a dedicated hardware, or as a virtualized function instantiated on an appropriate platform, such as a cloud infrastructure, and the like. It is to be understood in the context of this disclosure that the device(s) 700 can be implemented as a single device or as a plurality of devices with components and data distributed among them.

As illustrated, the device(s) 700 comprise a memory 702. In various embodiments, the memory 702 is volatile (including a component such as Random Access Memory (RAM)), non-volatile (including a component such as Read Only Memory (ROM), flash memory, etc.) or some combination of the two.

The memory 702 may include various components, such as an aggregation system 704, a confirmation system 706, and/or a control chart system (not shown). The aggregation system 704 and/or the confirmation system 706 may include methods, threads, processes, applications, or any other sort of executable instructions. The aggregation system 704, the confirmation system 706, and various other elements stored in the memory 702 can also include files and databases. In some cases, the memory 702 stores parameters of an individual. The control chart system may include methods, threads, processes, applications, or any other sort of executable instructions described herein.

The memory 702 may include various instructions which can be executed by at least one processor 708 to perform operations. For example, the processor(s) 708, when executing instructions of the aggregation system 704, may perform operations including aggregating parameters detected by multiple sensors into a single data transmission. The processor(s) 708, when executing instructions of the confirmation system 706, may perform operations including confirming whether the parameters are accurate. In some embodiments, the processor(s) 708 includes a Central Processing Unit (CPU), a Graphics Processing Unit (GPU), or both CPU and GPU, or other processing unit or component known in the art.

The device(s) 700 can also include additional data storage devices (removable and/or non-removable) such as, for example, magnetic disks, optical disks, or tape. Such additional storage is illustrated in FIG. 7 by removable storage 710 and non-removable storage 712. Tangible computer-readable media can include volatile and nonvolatile, removable and non-removable media implemented in any method or technology for storage of information, such as computer readable instructions, data structures, program modules, or other data. The memory 702, removable storage 710, and non-removable storage 712 are all examples of computer-readable storage media. Computer-readable storage media include, but are not limited to, RAM, ROM, EEPROM, flash memory or other memory technology, CD-ROM, Digital Versatile Discs (DVDs), Content-Addressable Memory (CAM), or other optical storage, magnetic cassettes, magnetic tape, magnetic disk storage or other magnetic storage devices, or any other medium which can be used to store the desired information and which can be accessed by the device(s) 700. Any such tangible computer-readable media can be part of the device(s) 700.

The device(s) 700 also can include input device(s) 714, such as a keypad, a cursor control, a touch-sensitive display, voice input device, etc., and output device(s) 716 such as a display (e.g., a liquid crystal display (LCD)), speakers, printers, etc. These devices are well known in the art and need not be discussed at length here. In particular implementations, a user can provide input to the device(s) 700 via a user interface associated with the input device(s) 714 and/or the output device(s) 716.

As illustrated in FIG. 7, the device(s) 700 can also include one or more wired or wireless transceiver(s) 718. For example, the transceiver(s) 718 can include a Network Interface Card (NIC), a network adapter, a LAN adapter, or a physical, virtual, or logical address to connect to the various base stations or networks contemplated herein, for example, or the various user devices and servers. To increase throughput when exchanging wireless data, the transceiver(s) 718 can utilize Multiple-Input/Multiple-Output (MIMO) technology. The transceiver(s) 718 can include any sort of wireless transceivers capable of engaging in wireless, Radio Frequency (RF) communication. The transceiver(s) 718 can also include other wireless modems, such as a modem for engaging in Wi-Fi, WiMAX, Bluetooth, or infrared communication.

## Claims

1. A patient monitoring system, comprising:
a support structure (106) configured to support an individual (102);
a first sensor configured to detect a first parameter (122) of the individual (102);
a second sensor configured to detect a second parameter (126) of the individual (102); and
**characterised by** a monitor (120) operably connected to the first sensor and the second sensor, the monitor (120) comprising:
a display configured to output at least one of the first parameter (122) and the second parameter (126);
an input device configured to receive a user input that confirms an accuracy of at least one of the first parameter (122) and the second parameter (126);
a transceiver configured to transmit, over a single transmission to an electronic medical record (EMR) server (130), data (128) identifying the individual (102) and indicating the first parameter (122) and the second parameter (126);
at least one processor communicatively coupled to the input device and the transceiver; and
memory storing instructions that, when executed by the at least one processor, cause the at least one processor to perform operations comprising:
confirming the first parameter (122) detected by the first sensor based on the second parameter (126) detected by the second sensor, and
based on determining that the second parameter (126) confirms the first parameter (122) and the user input, causing the transceiver to transmit the data (128) to the EMR system (130).

2. The patient monitoring system of claim 1, wherein the first parameter (122) comprises at least one of a heart rate of the individual (102), a blood pressure of the individual (102), a respiration rate of the individual (102), a capnograph of the individual (102), an oxygenation level of the individual (102), a temperature of the individual (102), a weight of the individual (102) on the support structure (106), or a presence of moisture between the support structure (106) and the individual (102),
wherein the second parameter (126) comprises at least one of the heart rate of the individual (102), the blood pressure of the individual (102), the respiration rate of the individual (102), the capnograph of the individual (102), the oxygenation level of the individual (102), the temperature of the individual (102), the weight of the individual (102) on the support structure (106), or the presence of moisture between the support structure (106) and the individual (102), and
wherein the first parameter (122) is different from the second parameter (126).

3. The patient monitoring system of either claim 1 or claim 2, wherein the transceiver is configured to transmit the data (128) in a single data stream.

4. The patient monitoring system of any preceding claim, further comprising:
a third sensor operably connected to the monitor (120) and configured to detect a third parameter of the individual (102), the operations further comprising determining, based on the third parameter, a position of the individual (102),
wherein causing the transceiver to transmit the data (128) is further based on the position of the individual (102).

5. The patient monitoring system of claim 4, wherein determining the position of the individual (102) comprises:
Determining that the individual (102) is at least one of supported by the support structure (106), sitting upright on the support structure (106), or laying down on the support structure (106).

6. The patient monitoring system of either claim 4 or claim 5, wherein:
the first sensor is configured to detect the first parameter (122) during a time interval,
the second sensor is configured to detect the second parameter (126) during the time interval, and
determining the position of the individual (102) comprises determining that the individual (102) has not sat up or laid down during the time interval.

7. The patient monitoring system of claim 1 wherein the first sensor is configured to detect a vital sign of an individual (102),
The second sensor is configured to detect a position of the individual (102), and
the memory stores instructions that, when executed by the at least one processor, cause the at least one processor to perform operations comprising:
determining that the position of the individual (102) confirms the accuracy of the vital sign; and
based on determining that the position of the individual (102) confirms the accuracy of the vital sign and that the user input confirms the accuracy of the vital sign, causing the transceiver to transmit the data (128) to the EMR system (130).

8. The patient monitoring system of claim 7, wherein the second sensor comprises at least one of a load cell (108) or a temperature sensor (122) integrated with the support structure (106).

9. The patient monitoring system of either claim 7 or claim 8, wherein the transceiver is configured to transmit the data identifying the individual and indicating the vital sign to an aggregator, the aggregator being configured to transmit the data in a single transmission to the EMR system (130).

10. The patient monitoring system of any one of claims 7 to 9, wherein determining that the position of the individual (102) confirms the accuracy of the vital sign comprises:
determining, based on the position of the individual (102), that the individual (102) is at least one of supported by the support structure (106), sitting upright on the support structure (106), or laying down on the support structure (106).

11. The patient monitoring system of any one of claims 7 to 10, wherein:
the first sensor is configured to detect the vital sign of the individual (102) during a time interval, and
determining that the position of the individual (102) confirms the accuracy of the vital sign comprises:
determining a movement of the individual (102) during the time interval based on the position of the individual (102), and
determining that the movement of the individual (102) during the time interval is less than a threshold movement.

12. The patient monitoring system of any one of claims 7 to 11, the vital sign being a first vital sign, the data being first data, the monitoring system further comprising:
a third sensor operably connected to the monitor (120) and configured to detect a second vital sign of the individual (102), wherein:
the display is configured to output the second vital sign with the first vital sign,
the user input further confirms an accuracy of the second vital sign,
the operations further comprise determining that the position of the individual (102) confirms the accuracy of the second vital sign, and
the transceiver is further configured to transmit, to the EMR system (130), second data indicating the second vital sign.

13. The patient monitoring system of any one of claims 7 to 12, wherein the transceiver is configured to transmit the first data and the second data to the EMR system (130) in a single transmission.

14. The patient monitoring system of any one of claims 7 to 13, wherein the first sensor is physically integrated with the support structure (106).

15. The patient monitoring system of any one of claims 1 to 14, wherein the memory stores instructions that, when executed by the at least one processor, cause the at least one processor to perform operations comprising:
identifying the first parameter (122) of the individual (102) detected during a time interval;
identifying the second parameter (126) of the individual (102) detected during the time interval;
determining, based on the second parameter (126), a position of the individual (102) during the time interval;
determining that the position of the individual (102) is substantially unchanged during the time interval;
based on determining that the position of the individual (102) is substantially unchanged, transmitting data identifying the individual (102) and indicating the first parameter (122) to the electronic medical record (EMR) system (130).

## Patentansprüche

1. Patientenüberwachungssystem, das Folgendes beinhaltet:
eine Trägeranordnung (106), die zum Tragen einer Person (102) konfiguriert ist;
einen ersten Sensor, der zum Erkennen eines ersten Parameters (122) der Person (102) konfiguriert ist;
einen zweiten Sensor, der zum Erkennen eines zweiten Parameters (126) der Person (102) konfiguriert ist; und
**gekennzeichnet durch** einen Monitor (120), der funktionell mit dem ersten Sensor und dem zweiten Sensor verbunden ist, wobei der Monitor (120) Folgendes beinhaltet:
eine Anzeige, die zum Ausgeben von wenigstens einem von dem ersten Parameter (122) und dem zweiten Parameter (126) konfiguriert ist;
eine Eingabevorrichtung, die zum Empfangen einer Benutzereingabe konfiguriert ist, welche eine Genauigkeit von wenigstens einem von dem ersten Parameter (122) und dem zweiten Parameter (126) bestätigt;
einen Transceiver, der zum Übertragen von Daten (128), die die Person (102) identifizieren und den ersten Parameter (122) und den zweiten Parameter (126) angeben, über eine einzelne Übertragung zu einem Server für elektronische Patientenakten (electronic medical record, EMR) (130) konfiguriert ist;
wenigstens einen Prozessor, der kommunikationsfähig mit der Eingabevorrichtung und dem Transceiver verbunden ist; und
Anweisungen zum Speichern in einem Speicher, die bei Ausführung durch den wenigstens einen Prozessor den wenigstens einen Prozessor zum Durchführen von Vorgängen veranlassen, die Folgendes beinhalten:
Bestätigen des von dem ersten Sensor erkannten ersten Parameters (122) auf Basis des von dem zweiten Sensor erkannten zweiten Parameters (126) und,
auf Basis des Bestimmens, dass der zweite Parameter (126) den ersten Parameter (122) und die Benutzereingabe bestätigt, Veranlassen des Transceivers zum Übertragen der Daten (128) zum EMR-System (130).

2. Patientenüberwachungssystem nach Anspruch 1, wobei der erste Parameter (122) wenigstens eines von einer Herzfrequenz der Person (102), einem Blutdruck der Person (102), einer Atemfrequenz der Person (102), einem Kapnogramm der Person (102), einem Sauerstoffgehalt der Person (102), einer Temperatur der Person (102), einem Gewicht der Person (102) auf der Tragkonstruktion (106) oder einem Vorhandensein von Feuchtigkeit zwischen der Tragkonstruktion (106) und der Person (102) beinhaltet,
wobei der zweite Parameter (126) wenigstens eines von der Herzfrequenz der Person (102), dem Blutdruck der Person (102), der Atemfrequenz der Person (102), dem Kapnogramm der Person (102), dem Sauerstoffgehalt der Person (102), der Temperatur der Person (102), dem Gewicht der Person (102) auf der Tragkonstruktion (106) oder dem Vorhandensein von Feuchtigkeit zwischen der Tragkonstruktion (106) und der Person (102) beinhaltet und
wobei der erste Parameter (122) vom zweiten Parameter (126) verschieden ist.

3. Patientenüberwachungssystem nach Anspruch 1 oder Anspruch 2, wobei der Transceiver zum Übertragen der Daten (128) in einem einzelnen Datenstrom konfiguriert ist.

4. Patientenüberwachungssystem nach einem der vorhergehenden Ansprüche, das ferner Folgendes beinhaltet:
einen dritten Sensor, der funktionell mit dem Monitor (120) verbunden ist und zum Erkennen eines dritten Parameters der Person (102) konfiguriert ist, wobei die Vorgänge ferner das Bestimmen einer Position der Person (102) auf Basis des dritten Parameters beinhalten,
wobei das Veranlassen des Transceivers zum Übertragen der Daten (128) ferner auf der Position der Person (102) basiert.

5. Patientenüberwachungssystem nach Anspruch 4, wobei das Bestimmen der Position der Person (102) Folgendes beinhaltet:
Bestimmen, dass die Person (102) wenigstens eines der folgenden ist: von der Tragkonstruktion (106) gestützt, aufrecht auf der Tragkonstruktion (106) sitzend oder auf der Tragkonstruktion (106) liegend.

6. Patientenüberwachungssystem nach Anspruch 4 oder Anspruch 5, wobei:
der erste Sensor zum Erkennen des ersten Parameters (122) während einer Zeitspanne konfiguriert ist,
der zweite Sensor zum Erkennen des zweiten Parameters (126) während der Zeitspanne konfiguriert ist und
Bestimmen der Position der Person (102) das Bestimmen, dass die Person (102) sich während der Zeitspanne nicht aufgesetzt oder hingelegt hat, beinhaltet.

7. Patientenüberwachungssystem nach Anspruch 1, wobei der erste Sensor zum Erkennen eines Vitalzeichens einer Person (102) konfiguriert ist;
der zweite Sensor zum Erkennen einer Position der Person (102) konfiguriert ist und
der Speicher Anweisungen speichert, die bei Ausführung durch den wenigstens einen Prozessor den wenigstens einen Prozessor zum Durchführen von Vorgängen veranlassen, die Folgendes beinhalten:
Bestimmen, dass die Position der Person (102) die Genauigkeit des Vitalzeichens bestätigt; und
Veranlassen des Transceivers, auf Basis des Bestimmens, dass die Position der Person (102) die Genauigkeit des Vitalzeichens bestätigt und dass die Benutzereingabe die Genauigkeit des Vitalzeichens bestätigt, zum Übertragen der Daten (128) zum EMR-System (130).

8. Patientenüberwachungssystem nach Anspruch 7, wobei der zweite Sensor wenigstens einen von einem Kraftaufnehmer (108) und einem Temperatursensor (122) beinhaltet, der in die Tragkonstruktion (106) integriert ist.

9. Patientenüberwachungssystem nach Anspruch 7 oder Anspruch 8, wobei der Transceiver zum Übertragen der Daten konfiguriert ist, die die Person identifizieren und das Vitalzeichen einem Aggregator angeben, wobei der Aggregator zum Übertragen der Daten zum EMR-System (130) in einer einzelnen Übertragung konfiguriert ist.

10. Patientenüberwachungssystem nach einem der Ansprüche 7 bis 9, wobei das Bestimmen, dass die Position der Person (102) die Genauigkeit des Vitalzeichens bestätigt, Folgendes beinhaltet:
Bestimmen, auf der Basis der Person (102), dass die Person (102) wenigstens eines der folgenden ist: von der Tragkonstruktion (106) gestützt, aufrecht auf der Tragkonstruktion (106) sitzend oder auf der Tragkonstruktion(106) liegend.

11. Patientenüberwachungssystem nach einem der Ansprüche 7 bis 10, wobei:
der erste Sensor zum Erkennen des Vitalzeichens der Person (102) während eines Zeitintervalls konfiguriert ist, und
Bestimmen, dass die Position der Person (102) die Genauigkeit der Vitalzeichen bestätigt, Folgendes beinhaltet:
Bestimmen einer Bewegung der Person (102) während der Zeitspanne auf Basis der Position der Person (102) und
Bestimmen, dass die Bewegung der Person (102) während der Zeitspanne kleiner als eine Schwellenbewegung ist.

12. Patientenüberwachungssystem nach einem der Ansprüche 7 bis 11, wobei das Vitalzeichen ein erstes Vitalzeichen ist, die Daten erste Daten sind, das Überwachungssystem ferner Folgendes beinhaltet:
einen dritten Sensor, der funktionell mit dem Monitor (120) verbunden ist und zum Erkennen eines zweiten Vitalzeichens der Person (102) konfiguriert ist, wobei:
die Anzeige zum Ausgeben des zweiten Vitalzeichens mit dem ersten Vitalzeichen konfiguriert ist,
die Benutzereingabe ferner eine Genauigkeit des zweiten Vitalzeichens bestätigt,
die Vorgänge ferner das Bestimmen, dass die Position der Person (102) die Genauigkeit des zweiten Vitalzeichens bestätigt, beinhalten und
der Transceiver ferner zum Übertragen von zweiten Daten, die das zweite Vitalzeichen angeben, zum EMR-System (130) konfiguriert ist.

13. Patientenüberwachungssystem nach einem der Ansprüche 7 bis 12, wobei der Transceiver zum Übertragen der ersten Daten und der zweiten Daten zum EMR-System (120) in einer einzelnen Übertragung konfiguriert ist.

14. Patientenüberwachungssystem nach einem der Ansprüche 7 bis 13, wobei der erste Sensor physisch in die Tragkonstruktion (106) integriert ist.

15. Patientenüberwachungssystem nach einem der Ansprüche 1 bis 14, wobei der Speicher Anweisungen speichert, die bei Ausführung durch den wenigstens einen Prozessor den wenigstens einen Prozessor zum Durchführen von Vorgängen veranlassen, die die folgenden beinhalten:
Identifizieren des während einer Zeitspanne erkannten ersten Parameters (122) der Person (102);
Identifizieren des während der Zeitspanne erkannten zweiten Parameters (126) der Person (102);
Bestimmen einer Position der Person (102) während der Zeitspanne auf Basis des zweiten Parameters (126);
Bestimmen, dass die Position der Person (102) während der Zeitspanne im Wesentlichen unverändert ist;
auf Basis des Bestimmens, dass die Position der Person (102) im Wesentlichen unverändert ist, Übertragen von Daten, die die Person (102) identifizieren und den ersten Parameter (122) dem System für elektronische Patientenakten (electronic medical record, EMR) (130) angeben.

## Revendications

1. Système de surveillance de patient, comprenant :
une structure de support (106) configurée pour soutenir un individu (102) ;
un premier capteur configuré pour détecter un premier paramètre (122) de l'individu (102) ;
un deuxième capteur configuré pour détecter un deuxième paramètre (126) de l'individu (102) ; et
**caractérisé par** un moniteur (120) connecté de manière opérationnelle au premier capteur et au deuxième capteur, le moniteur (120) comprenant :
un affichage configuré pour sortir au moins l'un du premier paramètre (122) et du deuxième paramètre (126) ;
un dispositif d'entrée configuré pour recevoir une entrée utilisateur qui confirme une précision d'au moins l'un du premier paramètre (122) et du deuxième paramètre (126) ;
un émetteur-récepteur configuré pour transmettre, en une seule transmission à un serveur d'enregistrement médical électronique (EMR) (130), des données (128) identifiant l'individu (102) et indiquant le premier paramètre (122) et le deuxième paramètre (126),
au moins un processeur couplé de manière communicative au dispositif d'entrée et à l'émetteur-récepteur ; et
une mémoire stockant des instructions qui, lorsque exécutées par le au moins un processeur, font que le au moins un processeur mette en oeuvre des opérations comprenant :
confirmer le premier paramètre (122) détecté par le premier capteur sur la base du deuxième paramètre (126) détecté par le deuxième capteur, et
sur la base de déterminer que le deuxième paramètre (126) confirme le premier paramètre (122) et l'entrée utilisateur, faire que l'émetteur-récepteur transmette les données (128) au système EMR (130).

2. Système de surveillance de patient selon la revendication 1, dans lequel le premier paramètre (122) comprend au moins l'un d'entre une fréquence cardiaque de l'individu (102), une pression artérielle de l'individu (102), une fréquence respiratoire de l'individu (102), une capnographie de l'individu (102), un niveau d'oxygénation de l'individu (102), une température de l'individu (102), un poids de l'individu (102) sur la structure de support (106), ou une présence d'humidité entre la structure de support (106) et l'individu (102),
dans lequel le deuxième paramètre (126) comprend au moins l'un d'entre la fréquence cardiaque de l'individu (102), la pression artérielle de l'individu (102), la fréquence respiratoire de l'individu (102), la capnographie de l'individu (102), le niveau d'oxygénation de l'individu (102), la température de l'individu (102), le poids de l'individu (102) sur la structure de support (106), ou la présence d'humidité entre la structure de support (106) et l'individu (102), et
dans lequel le premier paramètre (122) est différent du deuxième paramètre (126).

3. Système de surveillance de patient selon la revendication 1 ou la revendication 2, dans lequel l'émetteur-récepteur est configuré pour transmettre les données (128) en un seul flux de données.

4. Système de surveillance de patient selon l'une quelconque des revendications précédentes, comprenant en outre :
un troisième capteur connecté de manière opérationnelle au moniteur (120) et configuré pour détecter un troisième paramètre de l'individu (102), les opérations comprenant en outre, sur la base du troisième paramètre, une position de l'individu (102),
dans lequel faire que l'émetteur-récepteur transmette les données (128) est basé en outre sur la position de l'individu (102).

5. Système de surveillance de patient selon la revendication 4, dans lequel déterminer la position de l'individu (102) comprend :
déterminer que l'individu (102) est au moins l'un d'entre soutenu par la structure de support (106), assis droit sur la structure de support (106), ou couché sur la structure de support (106).

6. Système de surveillance de patient selon la revendication 4 ou la revendication 5, dans lequel :
le premier capteur est configuré pour détecter le premier paramètre (122) durant un intervalle de temps,
le deuxième capteur est configuré pour détecter le deuxième paramètre (126) durant l'intervalle de temps, et
déterminer la position de l'individu (102) comprend déterminer que l'individu (102) ne s'est pas assis ni couché durant l'intervalle de temps.

7. Système de surveillance de patient selon la revendication 1, dans lequel le premier capteur est configuré pour détecter un signe vital de l'individu (102),
le deuxième capteur est configuré pour détecter une position de l'individu (102), et
la mémoire stocke des instructions qui, lorsque exécutées par le au moins un processeur, font que le au moins un processeur effectue des opérations comprenant :
déterminer que la position de l'individu (102) confirme la précision du signe vital ; et
sur la base de déterminer que la position de l'individu (102) confirme la précision du signe vital et que l'entrée utilisateur confirme la précision du signe vital, faire que l'émetteur-récepteur transmette les données (128) au système EMR (130).

8. Système de surveillance de patient selon la revendication 7, dans lequel le deuxième capteur comprend au moins un dynamomètre (108) ou un capteur de température (122) intégré avec la structure de support (106).

9. Système de surveillance de patient selon la revendication 7 ou la revendication 8, dans lequel l'émetteur-récepteur est configuré pour transmettre les données identifiant l'individu et indiquant le signe vital à un agrégateur, l'agrégateur étant configuré pour transmettre les données en une seule transmission au système EMR (130).

10. Système de surveillance de patient selon l'une quelconque des revendications 7 à 9, dans lequel déterminer que la position de l'individu (102) confirme la précision du signe vital comprend :
déterminer sur la base de la position de l'individu (102) que l'individu (102) est au moins l'un d'entre soutenu par la structure de support (106), assis droit sur la structure de support (106) ou couché sur la structure de support (106).

11. Système de surveillance de patient selon l'une quelconque des revendications 7 à 10, dans lequel :
le premier capteur est configuré pour détecter le signe vital de l'individu (102) durant un intervalle de temps, et
déterminer que la position de l'individu (102) confirme la précision du signe vital comprend :
déterminer un mouvement de l'individu (102) durant l'intervalle de temps sur la base de la position de l'individu (102), et
déterminer que le mouvement de l'individu (102) durant l'intervalle de temps est moindre qu'un mouvement seuil.

12. Système de surveillance de patient selon l'une quelconque des revendications 7 à 11, le signe vital étant un premier signe vital, les données étant des premières données, le système de surveillance comprenant en outre :
un troisième capteur connecté de manière opérationnelle au moniteur (120) et configuré pour détecter un deuxième signe vital de l'individu (102), dans lequel :
l'affichage est configuré pour sortir le deuxième signe vital avec le premier signe vital,
l'entrée utilisateur confirme en outre une précision du deuxième signe vital,
les opérations comprennent en outre déterminer que la position de l'individu (102) confirme la précision du deuxième signe vital, et
l'émetteur-récepteur est configuré en outre pour transmettre, au système EMR (130), des deuxièmes données indiquant le deuxième signe vital.

13. Système de surveillance de patient selon l'une quelconque des revendications 7 à 12, dans lequel l'émetteur-récepteur est configuré pour transmettre les premières données et les deuxièmes données au système EMR (130) en une seule transmission.

14. Système de surveillance de patient selon l'une quelconque des revendications 7 à 13, dans lequel le premier capteur est physiquement intégré avec la structure de support (106).

15. Système de surveillance de patient selon l'une quelconque des revendications 1 à 14, dans lequel la mémoire stocke des instructions qui, lorsque exécutées par le au moins un processeur, font que le au moins un processeur effectue des opérations comprenant :
identifier le premier paramètre (122) de l'individu (102) détecté durant un intervalle de temps ;
identifier le deuxième paramètre (126) de l'individu (102) détecté durant l'intervalle de temps ;
déterminer, sur la base du deuxième paramètre (126), une position de l'individu (102) durant l'intervalle de temps ;
déterminer que la position de l'individu (102) est sensiblement inchangée durant l'intervalle de temps ;
sur la base de déterminer que la position de l'individu (102) est sensiblement inchangée, transmettre des données identifiant l'individu (102) et indiquant le premier paramètre (122) au système d'enregistrement médical électronique (EMR) (130).
